# EUROPÄISCHE PATENTSCHRIFT

(11) **EP 2 567 713 B1**
(45) Veröffentlichungstag und Bekanntmachung des Hinweises auf die Patenterteilung: **01.10.2014**
(21) Anmeldenummer: 12005891.2
(22) Anmeldetag: 16.08.2012
(51) Int. Cl.: A61L 2/10, A61L 9/20, C02F 1/32

(54) **Vorrichtung zur Entkeimung von Gasen oder Flüssigkeiten mittel UV**
Device for removing microbes from gases or liquids with UV
Dispositif de désinfection de gaz ou de liquides avec UV

(30) Priorität: 08.09.2011 DE 102011112994
(43) Veröffentlichungstag der Anmeldung: 13.03.2013
(73) Patentinhaber: SCHOTT AG, 55122 Mainz (DE)
(72) Erfinder: Blechschmidt, Jörg, Dr., 55270 Zornheim (DE); Bartsch, Reiner, 95643 Tirschenreuth (DE)
(74) Vertreter: Sawodny, Michael-Wolfgang

(56) Entgegenhaltungen:
- WO-A1-2009/013507
- DE-A1-102007 013 191
- DE-A1-102010 005 893
- US-A1- 2005 000 913
- US-B1- 7 270 748

## Beschreibung

Die Erfindung betrifft eine Vorrichtung zur Entkeimung von Gasen und/oder Flüssigkeiten.

Es ist bereits bekannt UV-Strahlung zur Behandlung, insbesondere Entkeimung oder Sterilisierung bzw. Desinfizierung, von Wasser, Luft oder Oberflächen einzusetzen. Relativ verbreitet ist bislang die Trinkwasseraufbereitung mit UV-Strahlung, wobei die Keimzahl im Wasser zuverlässig und in Abhängigkeit zur Dosis stark reduziert werden kann. Durch die UV-Strahlung werden Mikroorganismen, wie Krankheitserreger, insbesondere Bakterien oder Viren, inaktiviert.

Die UV-Entkeimung weist eine Reihe an Vorteilen gegenüber der herkömmlichen Desinfektion, basierend auf chemischen Verfahren, auf:
Bei der UV-Entkeimung handelt es sich um ein einfaches und schnell wirksames Verfahren, wobei die Entkeimung unmittelbar während der Belichtung des Mediums erfolgt. Ein großer Vorteil der UV-Entkeimung ist weiterhin, dass weder Geschmack, Geruch noch der pH-Wert des entkeimten Mediums beeinflusst wird, was einen wesentlichen Unterschied zur chemischen Behandlung von Trink- oder Prozesswasser darstellt. Im Gegensatz zum chemischen Desinfektionsverfahren sind zusätzliche Zugaben von desinfizierenden Mitteln nicht erforderlich, Wartung- und Überwachung von Dosieranlagen entfallen und spezielle Sicherheitsbestimmungen sind ebenfalls nicht notwendig. Ein weiterer Vorteil ist die Umweltfreundlichkeit, da keine Nebenreaktionen unter Bildung unerwünschter Verbindungen auftreten. Anders als übliche Desinfektionsmittel werden bei einer UV-Entkeimung keine mutationsbedingten Resistenzen entwickelt, wie dies beispielsweise bei krankenhausspezifischen Keimen (z.B. Antibiotika-Resistenz) häufig der Fall ist.

Die UV-Entkeimung ist auch im großen Maßstab möglich, beispielsweise in der kommunalen Trinkwasseraufbereitung. Auch ein Dauerbetrieb ist möglich, um die Keimzahl ständig gering zu halten.

Bei der UV-Entkeimung werden üblicherweise Quecksilberdampflampen eingesetzt, welche Strahlung bei einer Wellenlänge um 254 nm emittieren. Kürzere Wellenlängen unterhalb 200 nm sind so kurzwellig, dass sie durch molekularen Sauerstoff absorbiert werden, wodurch der molekulare Sauerstoff in freie Sauerstoffradikale gespalten wird, und mit weiteren Sauerstoffmolekülen zu Ozon weiterreagieren kann. Derartige kurzwellige UV-Strahlung wird u.a. zur Herstellung von hochreinem Wasser eingesetzt.

Aus dem Stand der Technik sind zahlreiche Vorschläge für die UV-Entkeimung bekannt geworden. Einige hiervon sollen nachfolgend erläutert werden:
So beschreibt die DE 38 37 905 A1 ein Verfahren und eine Vorrichtung zur Behandlung, insbesondere Entkeimung, von Flüssigkeiten und/oder Gasen mittels UV-Lichtquellen, wobei die Vorrichtung eine rohrförmige Reaktionskammer für das zu behandelnde Medium und mindestens zwei UV-Lichtquellen umfasst, wobei voneinander verschiedene UV-Lichtquellen vorgesehen sind, die jeweils unterschiedliche Wellenlängen abstrahlen und gemeinsam, in wählbaren Kombinationen miteinander oder getrennt im Wechsel betreibbar sind. Die UV-Lichtquellen befinden sich entweder in der Reaktionskammer und werden in das Medium eingetaucht und von diesem umspült oder werden außerhalb und auch mit Abstand von der Reaktionskammer angebracht.

Die DE 38 24 647 A1 bezieht sich auf eine Vorrichtung zur Bestrahlung von Medien mittels UV-Licht, bestehend aus einem vom Medium durchströmten Rohrkörper aus UV-durchlässigem Werkstoff und mindestens zwei außen achsparallel angeordneten UV-Lichtquellen mit Reflektoren, wobei die Lichtquellen UV-Flachstrahler darstellen, die einen länglich, flachovalen Querschnitt mit Breit- und Schmalseite aufweisen, wobei die Hauptachsen der UV-Lichtquellen jeweils auf den Mittelpunkt des Rohrkörperquerschnitts gerichtet sind. Die UV-Lichtquellen sind kranzförmig und achsparallel um den mediumdurchströmten Rohrkörper angeordnet. Gemäß einer Ausführungsform liegen die Flachstrahler mit der dem Rohrkörper zugewandten Schmalseite am Rohrkörper an.

Die US 5 133 932 offenbart eine Vorrichtung zur Sterilisierung von Blut und anderen Flüssigkeiten biologischen Ursprungs, indem ein für UV-Strahlung transparenter Behälter mit der zu sterilisierenden Flüssigkeit gedreht und gleichzeitig von außen mit UV-Strahlung bestrahlt wird. Der Behälter kann eine wellenförmige Oberfläche aufweisen. Die UV-Lichtquellen sind jedoch außerhalb des drehbaren Behälters angeordnet.

Weiterhin beschäftigt sich die DE 196 17 467 A1 mit einer Vorrichtung zum Entkeimen von Wasser mittels UV-C-Strahlen, wobei das Wasser durch ein Quarzglasrohr fließt und die ein oder mehreren UV-Strahler um das Quarzglasrohr herum angeordnet sind.

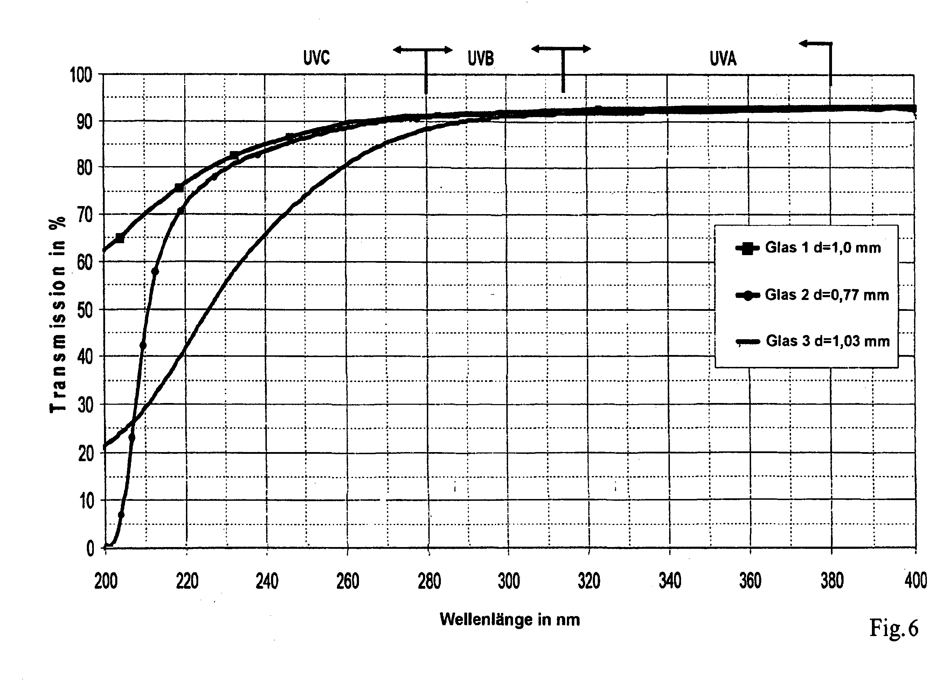

Die DE 10 2010 005 893 A1 offenbart eine Anlage zur Herstellung von Reinstwasser, umfassend wenigstens einen Einlass für das zu reinigende Wasser, eine Reinigungseinheit, eine UV-Bestrahlungseinrichtung mit wenigstens einer UV-Strahlen emittierenden Lichtquelle, die zur Bestrahlung des durch die UV-Bestrahlungseinrichtung strömenden Wassers ausgebildet ist, sowie einen Auslass. Die UV-Bestrahlungseinrichtungen, beispielsweise in Form von UV-LEDs, sind dabei entweder außerhalb des Leitungssystems 14 angeordnet (Fig. 6d) oder in die Wandung des Leitungssystems 14 integriert (Fig. 6a, 6b und 6c). Besonders bevorzugt ragt die UV-Bestrahlungseinrichtung zumindest teilweise in das strömende Wasser hinein.

Die WO 2009/013507 A1 betrifft eine Behandlungsvorrichtung zum mindestens teilweise Entkeimen eines Fluids, wie Wasser, umfassend eine Leitung zur Beförderung eines zu behandelnden Fluidstroms, eine Vielzahl von LEDs zur Emission von UV-Licht in das Fluid sowie einen Kontrollkreislauf zur Steuerung der LEDs mittels eine gepulsten Signals damit die Lichtquelle pulsiert. Die UV-LEDs sind hierbei derart angeordnet, dass der Fluidstrom unmittelbar über eine Oberfläche jeder Lichtquelle fließt. Der direkte Kontakt des Fluids mit der/den UV-Lichtquellen soll aufgrund der größeren Nähe von Lichtquelle zu behandelnder Flüssigkeit eine größere Behandlungseffizienz bewirken. Weiterhin soll ein Kühleffekt der UV-LEDs durch die Flüssigkeit vorliegen, so dass die LED mit höchster UV-Lichtintensität betreibbar sein soll, wodurch wiederum die Einsatzmöglichkeiten vergrößert und die Effizienz verbessert werden sollen.

Problematisch beim direkten Kontakt von UV-Lichtquelle und zu behandelndem Medium ist jedoch, dass die Oberfläche der UV-Lichtquelle gegenüber dem Medium beständig und gegen dieses abgedichtet sein muss. Jede UV-LED muss dabei für sich abgedichtet werden. Der Kühleffekt, beispielsweise einer Flüssigkeit, kann dann nicht mehr genutzt werden, wenn die Flüssigkeit selbst nicht kühl, sondern warm oder gar heiß ist. Dann bewirkt die heiße Flüssigkeit sogar eine zusätzlich Aufheizung der UV-LEDs und kann deren Haltbarkait deutlich beschränken.

Auch haben die hier geschilderten Behandlungssysteme den Nachteil, dass kein einfacher Austausch der UV-Lichtquelle erfolgen kann, da diese in direktem Kontakt mit dem zu behandelnden Medium steht. Eine Abschaltung des gesamten Systems wird daher nötig, worunter die Effizienz des Verfahrens leidet.

Die US 7 270 748 B1 beschreibt ein Wasserreinigungssystem für einen Wasserhahn unter Verwendung von UV-Strahlung. Ein Teil des Wasserdurchlaufs weist eine Vielzahl von UV-LEDs auf, die um die transparente Leitung herum angeordnet und in diese eingebettet vorliegen können.

Die US 2003/0170151 A1 offenbart ein System, in dem ein Fluid UV-Strahlung ausgesetzt wird, wobei das System eine Leitung zur Beförderung des Fluids umfasst, wobei die Leitung Ablenkeinrichtungen aufweist, um den Strom des Fluids gleichmäßiger zu gestalten. Die UV-Lichtquellen sind dabei in der Leitung angeordnet oder können auch auf der Ablenkeinrichtung vorgesehen werden. Dabei sind die UV-Lichtquellen wieder in direktem Kontakt mit dem zu behandelnden Fluid, woraus die bereits geschilderten Nachteile resultieren.

Die US 2010/0253207 A1 beschreibt eine flache Entladungslampe für Erzeugung von UV-Strahlung einer sehr speziellen Ausgestaltung, die u.a. auch zur Entkeimung/Sterilisierung von Luft, Wasser oder Oberflächen eingesetzt werden kann.

Aus dem Stand der Technik sind demnach im Wesentlichen zwei unterschiedliche Aufbaukonzepte für UV-Entkeimungsanlagen bekannt:
(a) Anlagen, bei denen die UV-Lichtquellen vom zu entkeimenden Medium umspült werden, und
(b) Anlagen, bei denen die Lichtquellen außerhalb des zu entkeimenden Mediums angeordnet sind.

Die vorliegende Erfindung beschäftigt sich mit Anlagen vom Typ (b) und stellt eine Verbesserung derartiger Anlagen des Typs (b) dar.

Nachteile der bekannten Anlagen vom Typ (b) sind geringe Effizienz und großer Bauraum. Die UV-Lichtquellen befinden sich bei diesen Anlagen außerhalb eines UV-durchlässigen Rohres, in dem das zu entkeimende Medium fließt. Um eine zur Entkeimung erforderliche Mindestbestrahlung im vollständig durchflossenen Innenraum zu garantieren, muss entweder eine Vielzahl von UV-Lichtquellen um das Rohr herum angeordnet werden oder über ein aufwändiges, vergleichsweise viel Platz benötigendes Reflektorsystem die UV-Strahlung weniger Lichtquellen in ausreichendem Maße verteilt werden. Um eine ausreichende Entkeimungsleistung zu erreichen, müssen jedoch insbesondere zwei Kriterien erfüllt werden: Die Strahlungsintensität muss über den gesamten zu entkeimenden Bereich ausreichend hoch sein, d.h. die Intensität der Strahlung zur Entkeimung sollte ein bestimmtes Strahlungsminimum nicht unterschreiten. Weiterhin sollte eine möglichst homogene Verteilung der Strahlung im zu entkeimenden Medium vorliegen. Aus dem Stand der Technik bekannte Anlagen haben jedoch typischerweise das Problem einer schlechten UV-Lichtausnutzung, verursacht durch Abschattungen, Mehrfachreflexionen und Verlustmechanismen, insbesondere an den Spiegeloberflächen des Reflektorsystems. Durch die in der Regel ungerichtete Abstrahlung der UV-Lichtquellen geht nur ein geringer Anteil der Strahlung auf direktem Weg durch das UV-durchlässige Rohr in das zu entkeimende Medium. Der übrige Anteil muss über Reflektoren in das Rohr gelenkt werden. Reflektormaterialien, wie etwa Aluminium, absorbieren jedoch einen signifikanten Teil der UV-Strahlung, beispielsweise bei Aluminium sind dies ca. 15% bei einer Wellenlänge von 254 nm. Dieser Anteil der UV-Strahlung geht verloren und steht für die Entkeimung nicht mehr zur Verfügung.

Weiterhin benötigen die bekannten Anordnungen von UV-Lichtquellen und Reflektoren viel Platz und eignen sich daher nur bedingt für Anwendungen mit geringem Bauraum.

Demnach liegt der vorliegenden Erfindung die Aufgabe zugrunde, eine Vorrichtung bereitzustellen, welche die Nachteile des Standes der Technik vermeidet und eine effektive Entkeimung von Flüssigkeiten oder Gasen mittels UV-Strahlung unter Beibehaltung der bekannten Vorteile einer UV-Entkeimung bereitstellt, wobei eine bessere Ausnutzung der durch die Lichtquellen erzeugten UV-Strahlung erzielt wird. Weiterhin soll die erfindungsgemäße Vorrichtung eine höhere Kompaktheit hinsichtlich der Bauweise ermöglichen.

Erfindungsgemäß wird die Aufgabe der vorliegenden Erfindung durch eine Vorrichtung zur Entkeimung von Gasen oder Flüssigkeiten gelöst, umfassend die Merkmale des Anspruchs 1.

Die Geometrie der Erfindung basiert daher auf einer Anordnung mindestens einer UV-Lichtquelle, die zumindest teilweise von der Glaswand des Rohrs umgeben und in dieser angeordnet ist. Die Form der Glaswand des Rohrs ist dabei derart ausgestaltet, dass die Glaswand die UV-Lichtquelle zumindest teilweise, bevorzugt vollständig aufnehmen und unterzubringen kann. Zum Vorsehen der einen oder mehreren UV-Lichtquellen werden daher Einbuchtungen der Glasrohrwand bereitgestellt. Es liegt somit eine klare räumliche Trennung von UV-Lichtquelle und zu behandelndem Medium vor, das zu entkeimende Gas und/oder die zu entkeimende Flüssigkeit befindet sich nicht in direktem Kontakt mit der UV-Lichtquelle.

Eine "Einbuchtung" soll im Rahmen der vorliegenden Erfindung eine Einstülpung der Glaswand des Glasrohres vorbestimmter Größe darstellen, in der zumindest eine UV-Lichtquelle angeordnet ist. Dabei sind die Rohrinnenwand und die Rohraußenwand gleichzeitig an derselben Stelle nach Innen gestülpt und bilden zusammen einen zur Rohraußenseite offenen Hohlraum aus, in den die mindestens eine UV-Lichtquelle aufgenommen wird. Der von der Einbuchtung gebildete Hohlraum ist zur Rohraußenseite offen, um den Zugang zur UV-Lichtquelle zu erleichtern. Hierdurch kann die UV-Lichtquelle in einfacher Weise an die elektrischen Anschlüsse angebracht und wieder von diesen abgekoppelt werden, so dass die UV-Lichtquellen ohne weiteres ausgetauscht werden können. Die erfindungsgemäßen Einbuchtungen erstrecken sich daher zumindest teilweise in den Innenraum des Glasrohrs.

Erfindungsgemäß können auch Aussparungen im Glasrohr vorhanden sein. Eine "Aussparung" bedeutet erfindungsgemäß eine Vertiefung vorbestimmter Größe, die entweder in der Rohraußenwand oder der Rohrinnenwand des Glasrohrs vorgesehen ist. Bei Vorsehen einer Aussparung wird Material abgetragen: Wenn beispielsweise in der Rohrinnenwand eine oder mehrere Aussparungen vorgesehen sind, wird hierfür Material entfernt, um die Aussparungen auszubilden, jedoch bleibt die Rohraußenwand hiervon unberührt. Wenn beispielsweise in der Rohraußenwand eine oder mehrere Aussparungen vorgesehen sind, wird hierfür Material entfernt, um die Aussparungen auszubilden, jedoch bleibt die Rohrinnenwand hiervon unberührt. Die Aussparungen erstrecken sich im Gegensatz zu den erfindungsgemäß vorgesehenen Einbuchtungen nicht in den Innenraum des Glasrohrs, dieser bleibt bei Vorsehen von Aussparungen unberührt.

Die gegebenenfalls zusätzlich zu den erfindungsgemäßen Einbuchtungen vorliegenden Aussparungen können eine oder mehrere UV-Lichtquellen aufnehmen. Bevorzugt dienen die optionalen Aussparungen jedoch für andere Funktionen und enthalten keine UV-Lichtquellen. Beispielsweise können die Aussparungen einen Sensor enthalten oder andere technische Einrichtungen, welche für die Vorrichtung der Erfindung eingesetzt werden können. Nach einer weiteren bevorzugten Ausführungsform können auch nur Einbuchtungen im Glasrohr vorgesehen sein, Aussparungen liegen dann nicht vor.

Die Anzahl der verwendeten UV-Lichtquellen kann erfindungsgemäß relativ beliebig ausgewählt werden. Es ist zumindest eine UV-Lichtquelle vorhanden. Es können auch mindestens 2 UV-Lichtquellen vorliegen. Beispielhafte Ausführungsformen umfassen 1 bis 8 UV-Lichtquellen, bevorzugter 1 bis 6 UV-Lichtquellen, insbesondere 1 bis 5 UV-Lichtquellen, ganz besonders bevorzugt sind 1 bis 4 oder 1 bis 3 UV-Lichtquellen im Glasrohr. Wenn relativ stark strahlende UV-Lichtquellen, wie übliche UV-Stablampen, zum Einsatz kommen, ist es aus Kostengründen bevorzugt, eine möglichst geringe Anzahl an UV-Lichtquellen einzusetzen, bevorzugt 1 bis maximal 3 UV-Lampen. Wenn relativ schwach strahlende UV-Lampen zum Einsatz kommen, wie UV-LEDs, so kann erfindungsgemäß auch eine deutlich größere Anzahl an UV-Lichtquellen eingesetzt werden, beispielsweise 100 UV-LEDs oder mehr. In jedem Fall sollte eine vordefinierte Mindeststrahlungsintensität nicht unterschritten werden und eine möglichst gleichmäßige Verteilung des UV-Lichts vorliegen, um eine ausreichende Entkeimung zu gewährleisten.

Die Anordnung der UV-Lichtquellen erfolgt, je nach der gewählten Anzahl, derart, dass vorzugsweise eine möglichst gleichmäßige Verteilung der Lichtquellen über den gesamten Rohrumfang vorliegt. Besonders bevorzugt sind daher symmetrische Anordnungen, wie z.B. achsen- bzw. spiegelsymmetrische, rotationssymmetrische oder punktsymmetrische Anordnungen. Bei einer nicht symmetrischen Anordnung der UV-Lichtquellen, können Stellen im zu entkeimenden Medium resultieren, an die deutlich weniger UV-Licht gelangt, was vermieden werden sollte. Bei Vorsehen von 3 UV-Lichtquellen werden die UV-Lichtquellen daher vorzugsweise derart angeordnet, dass diese ein gleichseitiges Dreieck aufspannen (gedachte Verbindungslinien zwischen den einzelnen UV-Lichtquellen als Ecken stellen ein gleichseitiges Dreieck dar). Bei 4 UV-Lichtquellen werden diese vorzugsweise an den Ecken eines gedachten Quadrats angeordnet. Bei 5 UV-Lichtquellen befinden sich diese vorzugsweise in den Ecken eines Fünfecks mit gleichlangen Seiten etc.

Die Auswahl einer geeigneten Anordnung der UV-Lichtquellen hängt neben der Anzahl, Größe und Form der UV-Lichtquellen auch von der gewählten Form, Größe und dem Querschnitt des Glasrohrs ab. Ein Fachmann aus dem Stand der Technik kann ohne weiteres eine geeignete, vorzugsweise möglichst symmetrische Anordnung der UV-Lichtquellen für jeden Rohrtyp auswählen.

Die Anzahl der Einbuchtungen stimmt vorzugsweise mit der Anzahl der verwendeten UV-Lichtquellen überein. In Einzelfällen können aber auch mehr Einbuchtungen vorliegen als UV-Lichtquellen oder es können mehrere UV-Lichtquellen in einer Einbuchtung vorliegen.

Weiterhin bevorzugt ist jeweils nur eine UV-Lichtquelle in einer Einbuchtung vorgesehen. Dies führt zu einer besseren Ausnutzung der UV-Strahlung unter Vermeidung einer gegenseitigen Abschattung der UV-Lichtquellen. Bei Verwendung von UV-LEDs sind im Gegensatz hierzu mehrere, möglicherweise sogar eine große Anzahl an UV-Lichtquellen in einer Einbuchtung angeordnet. Dies liegt daran, dass LEDs gerichtet abstrahlen und so eine gegenseitige Abschattung vermieden werden kann.

Die erfindungsgemäße Anordnung hat besonders bei nicht vollständig UV-transparenten Rohrmaterialien, insbesondere bei den eingesetzten UV-transparenten Gläsern Vorteile. Da Glasrohre von UV-transparenten Gläsern bei einer Wellenlänge von 254 nm eine Absorption von >10% auf 1 mm Materialdicke zeigen können, ist es bei der erfindungsgemäßen Vorrichtung besonders vorteilhaft, dass das Licht nur kurze Wege durch das Glas zurücklegt, und nicht bereits im Rohrmaterial zu hohen Anteilen absorbiert wird.

Der Begriff "UV-transparent" bedeutet, dass das erfindungsgemäß eingesetzte Glas des Rohrs eine hohe UV-Transmission aufweist, was bedeutet, dass eine UV-Transmission von mindestens 75 % bei einer Wellenlänge von 254 nm und einer Schichtdicke des Glases von 1 mm vorliegt. Nach einer besonders bevorzugten Ausführungsform zeigt das Glas des Rohrs eine Transmission bei einer Schichtdicke von 1 mm im UV-Bereich, die bei 200 nm < 0,5% liegt und bei 254 nm > 75% liegt. Noch bevorzugter wird eine Transmission bei einer Schichtdicke von 1 mm im UV-Bereich bei 200 nm < 0,3% und bei 254 nm > 80 % erhalten.

Die erfindungsgemäße Lösung der Bereitstellung der UV-Lichtquellen in Einbuchtungen zeichnet sich dadurch aus, dass die UV-Lichtquellen in Richtung zum Innenraum des Rohrs versetzt sind. Es resultiert ein hoher Anteil an UV-Strahlung, der direkt durch das Glasrohr in das Medium geführt wird, ohne vorher unter Verlusten reflektiert zu werden. Durch die erfindungsgemäß bereitgestellte Geometrie wird weiterhin eine homogenere Lichtverteilung im Rohrinnenraum erzielt, wodurch die Entkeimungsleistung erhöht wird. Durch die erfindungsgemäße Anordnung wird zudem eine hohe Kompaktheit des Systems zur Verfügung gestellt.

So gelangt bei der erfindungsgemäßen Vorrichtung mehr UV-Strahlung an/in das Medium, als bei einer herkömmlichen Anordnung, wo sich die UV-Lichtquellen außerhalb des das zu entkeimende Medium führenden Rohrs befinden. Im Gegensatz zu den oben beschriebenen Anlagen gemäß Typ (a), die in der Regel auf einem sehr komplexen Systemaufbau beruhen, ist die erfindungsgemäße Vorrichtung relativ einfach aufgebaut. Insbesondere muss in Systemen vom Typ (a) die UV-Lichtquelle von außen zugänglich sein, was zusätzlichen konstruktiven Aufwand bedeutet, womit wiederum hohe Kosten verbunden sind. Die erfindungsgemäße Vorrichtung ermöglicht demgegenüber einen einfachen Zugang zur UV-Lichtquelle, so dass diese ohne weiteres ausgetauscht werden kann. Aufgrund des erfindungsgemäß einfachen Systemaufbaus kann ein nahezu ungestörter Durchfluss des Mediums durch das Glasrohr erfolgen.

Gemäß einer vorteilhaften Ausführungsform der Erfindung kann die Form der Einbuchtungen an die Form der verwendeten UV-Lichtquellen angepasst sein. Die Einbuchtung ist zweckmäßigerweise so ausgestaltet, dass diese die UV-Lichtquelle zumindest teilweise, bevorzugt vollständig, in sich aufnehmen kann. Die auf die Lichtquelle angepasste spezielle Form der Einbuchtung dient zur Optimierung der Lichtverteilung, so dass alle Bereiche innerhalb des Rohres möglichst eine ausreichende Bestrahlungsstärke erhalten. Dies spielt insbesondere am inneren Rohrrand, mittig zwischen einzelnen Lichtquellen eine Rolle, wo ansonsten nur geringe Strahlungsintensität vorliegen würde.

Nach einer bevorzugten Ausführungsform der Erfindung wird die mindestens eine UV-Lichtquelle in der mindestens einen Einbuchtung derart angeordnet, dass sich die mindestens eine UV-Lichtquelle zumindest teilweise, besonders bevorzugt vollständig, innerhalb der Rohrinnenwand des UV-transparenten Glasrohrs befindet, wenn die Rohrinnenwand ohne Einbuchtungen vorliegen würde. Unter "Rohrinnenwand" wird hier die Innenseite des Rohrs verstanden, wie diese ohne Einbuchtungen aussehen würde, d.h. wenn keine Einbuchtungen im Glasrohr vorhanden wären.

Nach der weiteren bevorzugten Ausführungsform, für den Falle, dass das Glasrohr einen runden Querschnitt aufweist, reichen die Einbuchtungen vorzugsweise so weit in den Innenraum des Glasrohrs, dass sich die darin befindlichen UV-Lichtquellen zumindest teilweise, besonders bevorzugt vollständig, innerhalb des Innenradius des Rohrs befinden. Unter "Rohrinnenradius" wird hier die Innenseite des Rohrs verstanden, wie diese ohne Einbuchtungen aussehen würde, d.h. wenn keine Einbuchtungen im Glasrohr vorhanden wären.

Durch diese erfindungsgemäße Geometrie kann ein Großteil der Strahlung (mindestens 180°-Abstrahlwinkel) auf direktem Weg durch die Glaswand in den Innenbereich gelangen und geht nicht durch Rückreflektionen in die Lichtquellen und/oder anderweitige Absorption beispielsweise am Reflektor verloren. Besonders bevorzugt sind Anordnungen, bei denen die UV-Lichtquellen derart angeordnet werden, dass sich diese innerhalb der Rohrinnenwand befinden, wie oben definiert, so dass eine besonders günstige Strahlungsverteilung resultiert, bei der der überwiegende Teil des UV-Lichts auf direktem Weg durch das Glas in den Innenraum gelenkt wird. Im Vergleich zu herkömmlichen Anordnungen gelingt es erfindungsgemäß daher, dass nur noch ein geringer Strahlungsanteil über Reflektoren zurückreflektiert wird. Es resultiert somit eine höhere Gesamteffizienz des Systems.

Die Einbuchtungen sind erfindungsgemäß bevorzugt lokal begrenzt im Glasrohr vorgesehen, d.h. die Einbuchtungen weisen eine vordefinierte Form und Größe auf; diese liegen vorzugsweise nur im Bereich einer UV-Lichtquelle oder einer Gruppe von UV-Lichtquellen vor und dehnen sich nicht weiter längs der Rohrachse aus. Es kann aber auch vorteilhaft sein, beispielsweise aus produktionstechnischen Gründen, eine Einbuchtung vorzusehen, die größer ist als die enthaltene UV-Lichtquelle. Die Einbuchtungen können beispielsweise auch umlaufend um das Rohr vorliegen.

Bevorzugt ist jeweils nur eine UV-Lichtquelle oder eine Gruppe von UV-Lichtquellen in einer Einbuchtung vorgesehen. Wenn erfindungsgemäß nur Einbuchtungen im Glasrohr vorgesehen sind, kann dies für das eingesetzte Herstellungsverfahren besonders zweckmäßig sein; dies wird noch im Einzelnen beschrieben.

Eine weitere bevorzugte Komponente in der erfindungsgemäßen Vorrichtung ist ein Reflektor, der außerhalb des Rohres angeordnet wird, um das nach außen abgestrahlte UV-Licht wieder in das Rohr hinein zu reflektieren. Die Reflektoren dienen zudem dazu, durch eine gezielte Reflektorgeometrie den Anteil der in die Lichtquelle zurückreflektierten UV-Strahlung auf ein Minimum zu reduzieren, bzw. aus dem Medium, insbesondere auf der gegenüberliegenden Seite, austretendes UV-Licht in das Medium zurück zu reflektieren.

Die Art, Form, Größe und der Aufbau des Reflektors sind erfindungsgemäß nicht weiter beschränkt. Ein Reflektor kann jede Art von Bauteil sein, umfassend eine Oberfläche zur Reflektion von Licht. Der Reflektor kann in verschiedenen Varianten ausgeführt werden. Der Reflektor kann beispielsweise aus flexiblem oder starrem bzw. festem Material aufgebaut sein. Bevorzugt weist der Reflektor eine Form und Größe auf, die an die Form und Größe der erfindungsgemäßen Vorrichtung, insbesondere das UV-transparente Glasrohr mit mindestens einer UV-Lichtquelle, angepasst ist.

Vorteilhafterweise kann der Reflektor beispielsweise um das Glasrohr herum angeordnet sein und dieses vollständig einhüllen, d.h. der Reflektor ist um die gesamte erfindungsgemäße Vorrichtung herum angeordnet. Der Reflektor kann hierfür vorzugsweise die Form eines Rohrs aufweisen, beispielsweise ein Rohr aus Aluminium, Edelstahl oder einem anderen Material, kann gegebenenfalls mit einer entsprechenden reflektierenden Beschichtung versehen sein und einen größeren Durchmesser als das Glasrohr aufweisen, das der Reflektor vollumfänglich umschließt. Dabei kann die Form und insbesondere der Querschnitt des Reflektorrohrs der Form bzw. dem Querschnitt des Glasrohres ähnlich sein. Das Reflektorrohr kann gleichzeitig als Schutz für das Glasrohr dienen.

Nach einer weiteren besonders bevorzugten Ausführungsform der Erfindung ist ein Reflektor unmittelbar auf dem Glasrohr angebracht, wobei gegebenenfalls im Bereich der Einbuchtungen kein Reflektor vorgesehen ist. Beispielsweise kann der Reflektor auf der Rohraußenwand aufgebracht sein, ausgenommen in dem Bereich, wo die Einbuchtungen und gegebenenfalls Aussparungen vorliegen, bevorzugt in Form einer UV-reflektierenden Beschichtung auf der Rohraußenwand.

Nach einer weiteren bevorzugten Ausführungsform der Erfindung kann der Reflektor auch auf der Innenseite des Glasrohrs angebracht sein, beispielsweise kann eine UV-reflektierende Beschichtung auf der Rohrinnenwand vorgesehen werden. Dabei sind die Einbuchtungen für die UV-Lichtquellen wieder ausgespart. Das reflektierte Licht wird hierbei nicht durch die Restabsorption des Glases abgeschwächt.

Der Reflektor kann sich auch aus mehreren verschiedenen Komponenten zusammensetzen, z.B. einzelnen Reflektoren, die vorzugsweise hinter den UV-Lichtquellen angeordnet sind. Bevorzugt ist dann jeder UV-Lichtquelle oder einer Gruppe von UV-Lichtquellen ein Reflektor zugeordnet, um eine möglichst hohe Strahlungsenergie für die Bestrahlung des im Rohr fließenden oder strömenden Mediums zur Verfügung zu stellen. Die einzelnen den jeweiligen UV-Lichtquellen zugeordneten Reflektoren sind in Form und Gestalt vorzugsweise den Formen eines Kegelschnitts angenähert, beispielsweise parabolisch oder elliptisch.

Es kann auch eine Kombination von unterschiedlichen Reflektoren vorliegen. Bevorzugt ist auf der Außen- oder der Innenseite des Glasrohrs ein Reflektor vorgesehen und zusätzlich an jeder UV-Lichtquelle ebenfalls ein Reflektor vorgesehen. Beispielsweise kann ein Reflektor separat auf dem Glasrohr außen oder innen angeordnet sein oder unmittelbar auf das Glasrohr aufgebracht sein, ausgenommen im Bereich der Einbuchtungen, wo für jede UV-Lichtquelle ein zusätzlicher Reflektor vorgesehen sein kann. Nach einer besonders bevorzugten Ausführungsform der Erfindung ist beispielsweise auf der Rohraußenwand oder der Rohrinnenwand der erfindungsgemäßen Vorrichtung eine UV-reflektierende Beschichtung vorgesehen und in dem Bereich, wo sich die Einbuchtungen und gegebenenfalls Aussparungen befinden und die Strahlung in den Innenraum gelangen soll, liegt keine Beschichtung vor, da hier die Oberfläche unterbrochen ist, so dass jeweils separate Reflektoren vorgesehen sein können.

In der erfindungsgemäßen Vorrichtung ist daher eine einfache Reflektorgeometrie möglich, wobei der Reflektor separat vom Glasrohr - außen oder innen - oder auf das Glasrohr aufgebracht, unter Auslassung der UV-Lichtquellen, angeordnet sein kann.

Auch Form, Größe oder der Querschnitt des Glasrohrs ist erfindungsgemäß nicht besonders beschränkt. Der Querschnitt des Rohrs ist beliebig wählbar, sofern die baulichen Gegebenheiten für die beabsichtigte Verwendung dies ermöglichen. Der Querschnitt des Rohrs ist beispielsweise ausgewählt aus rund, oval, eckig, insbesondere 3-, 4-, 5-, 6-, 7- oder 8-eckig, bevorzugt rund oder oval. Beispielsweise kann der Rohrquerschnitt eine fünfeckige Grundkontur mit Einbuchtungen für die Lampen an den fünf Ecken darstellen oder der Rohrquerschnitt kann eine elliptische Grundkontur mit Einbuchtungen an den spitzeren Rundungen aufweisen oder der Rohrquerschnitt ist rund mit vorzugsweise gleichmäßiger Verteilung der UV-Lichtquellen über den gesamten Rohrumfang.

Besonders bevorzugt ist erfindungsgemäß ein rundes Rohr oder ein Rohr, das eine im Wesentlichen runde Grundkontur aufweist. Wenn Einbuchtungen in einem Rohr mit runder Grundkontur in der Glaswand vorliegen, so kann die Rohrinnenwand eine runde Innenkontur aufweisen und die Rohraußenwand kann ebenfalls eine runde Außenkontur aufweisen, die nur durch die vorliegenden Einbuchtungen unterbrochen wird.

Eine im Wesentlichen runde Grundkontur ist erfindungsgemäß möglichst breit gefasst zu verstehen, wonach ein Rohr im Querschnitt beispielsweise auch eine stern- oder wellenförmige Gestalt aufweisen kann, die sich von der runden Grundform ableitet, die noch vorhanden ist, wenn man sich die zusätzlichen Strukturen wegdenkt. Der Rohrquerschnitt kann selbstverständlich auch eine exakt runde Außen- und Innenkontur aufweisen, d.h. mit einem vordefinierten Innen- und Außenradius.

Die Einbuchtungen können erfindungsgemäß beliebige Form und Größe aufweisen, können beispielsweise spitz zulaufend und/oder abgerundet ausgebildet sein. Bevorzugt sind jedoch runde oder abgerundete Formen, die an die Form der UV-Lichtquelle angepasst sind. Weiterhin bevorzugt sind Formen, die eine gezielte Lenkung des UV-Lichts in den Glasinnenraum ermöglichen. Besonders bevorzugt sind sämtliche im Glasrohr vorhandenen Einbuchtungen von gleicher Form und Größe.

Die erfindungsgemäß eingesetzten UV-Lichtquellen sind im Rahmen der Erfindung ebenfalls nicht besonders beschränkt, es kann jede Art an bekannten UV-Lichtquellen zum Einsatz kommen, wobei üblicherweise UV-Strahlung bei einer Wellenlänge von 253,7 nm eingesetzt wird. Dies stellt die Hauptemissionswellenlänge von Niederdruck-UV-Lampen und ein wesentliches Strahlungsmaximum anderer UV-Lampen dar. Eingesetzt werden daher beispielsweise Mitteldruck-, Hochdruck- oder Niederdruck-UV-Lampen, bevorzugt Quecksilberdampf-Mitteldruck-, -Hochdruck- oder Niederdruck-Lampen, welche Strahlung bei einer Wellenlänge um 254 nm emittieren. Niederdruck-UV-Lampen, insbesondere Niederdruck-Quecksilberdampflampen sind besonders bevorzugt. Nach einer weiteren Ausführungsform der Erfindung sind UV-Lichtquellen in Form von CCL (Cold Cathode Lamp) besonders bevorzugt. Diese basieren auf der bewährten CCFL-Technologie (Cold Cathode Fluorescent Lamp) wobei auf die Fluoreszenz-Beschichtung verzichtet wird und können heute bereits frei am Markt bezogen werden. Erfindungsgemäß können auch UV-LEDs zum Einsatz kommen. Beim Einsatz von UV-LEDs kann eine höhere Wellenlänge im Bereich von 270nm gewählt werden, bei der einerseits die Entkeimungswirkung größer ist. Andererseits besitzen typische UV-transparente Gläser bei diesen Wellenlängen eine höhere Transmission, was die Effizienz zusätzlich erhöht.

Generell zeichnen sich Rohre dadurch aus, dass deren Rohrwand eine bestimmte Gestalt in Form einer Kontur aufweist, die sich in Richtung der Längsachse des Rohres in bestimmter Länge erstreckt. Die Kontur (Profil) wird in der vorliegenden Erfindung bestimmt durch Einbuchtungen, die vorzugsweise jeweils im Wesentlichen parallel zur Rohrachse verlaufen. Eine Kontur kann auf der Außenseite eines Rohres als sog. Außenkontur und/oder auf der Innenseite eines Rohres als sog. Innenkontur vorliegen. Die Innen- und Außenkontur können derart kombiniert und aufeinander abgestimmt sein, dass die Wandstärke des Rohres längs des Rohrumfangs konstant ist oder variiert.

Erfindungsgemäß ist bei Vorsehen von ein oder mehreren Einbuchtungen in der Rohrwand die Wandstärke des Rohres vorzugsweise längs des Rohrumfangs konstant. Bei einer erfindungsgemäßen Einbuchtung stimmt die Außenkontur mit der Innenkontur überein, die Außenkontur weist immer dann eine Einbuchtung auf, wenn die Innenkontur auch eine Einbuchtung aufweist.

Die Innen- und Außenkontur können auch derart kombiniert und aufeinander abgestimmt sein, dass die Wandstärke des Rohres längs des Rohrumfangs variiert wird.

Generell kann die Kontur eine regelmäßige Form oder eine unregelmäßige Form aufweisen. Aufgrund der bevorzugten symmetrischen Anordnung der UV-Lichtquellen ist eine regelmäßige Form bevorzugt. Die Einbuchtung kann z.B. eine Wellenform besitzen oder eine abgerundete, recheckige oder Zackenform aufweisen. Kombinationen von unterschiedlichen Formen sind möglich.

Die erfindungsgemäß vorgesehenen Einbuchtungen und gegebenenfalls Aussparungen in einem Glasrohr beliebiger Form können gemäß dem Wissen des Fachmanns im Bereich der Glastechnologie ohne weiteres hergestellt werden. Hierbei handelt es sich um die sog. Konturgebung, auch bezeichnet als Profilierung der Außenseite und/oder Innenseite eines Glasrohres.

Die Konturen in Form von Einbuchtungen und gegebenenfalls Aussparungen können bereits während der Glasherstellung erzeugt werden. In einem besonders bevorzugten Verfahren werden die Konturen direkt im Heißformgebungsverfahren in das Glasrohr eingebracht. Das Einbringen von Konturen bzw. Profilen in ein Glasrohr sind in der DE 10 2004 018 148 A1 (Conturax®-Technologie) beschrieben, deren Offenbarungsgehalt vollumfänglich in die vorliegende Anmeldung mit aufgenommen wird.

Bei dem aus der DE 10 2004 018 148 A1 bekannten Verfahren wird ein kontinuierliches Rohrziehverfahren verwendet, um kalibrierte runde oder profilierte Glasrohre mit einem vorgegebenen Innenprofil und/oder einem vorgegebenen Außenprofil herzustellen. Hierbei wird die Glasschmelze während des Ziehvorganges über einen speziellen Profilformungskörper gezogen. Das Verfahren kann beispielsweise auf das bekannte Down-Draw- oder Vello-Verfahren und das Danner-Verfahren angewendet werden.

Bei diesen kontinuierlichen Rohrziehverfahren sind neben der Viskosität des Glases die Parameter Innendruck, Glasdurchsatz, Ziehgeschwindigkeit und Abmessungen der Formwerkzeuge entscheidend, wobei alle Parameter entsprechend aufeinander abgestimmt werden. Rohrdurchmesser und Wanddicke sind unabhängig voneinander wählbar. Die Ziehgeschwindigkeit ist dabei für eine vorgegebene Rohrabmessung (Außendurchmesser und Wanddicke) aufgrund des Kontinuitätsgesetzes mit dem Glasdurchsatz korreliert.

Bei Herstellung von Glasrohren gemäß der DE 10 2004 018 148 A1 kann das Vorsehen einer größeren Aussparung in der Außenkontur des Glasrohrs bei entsprechender Verfahrensführung auch zu einer Veränderung der Innenkontur führen, so dass eine Einbuchtung erhalten wird. So können bei entsprechender Anwendung des Conturax®-Verfahrens Einbuchtungen und gegebenenfalls auch Aussparungen in einem Glasrohr hergestellt werden.

Mit der Conturax®-Technologie kann demnach direkt ein Glasrohr mit den erforderlichen Einbuchtungen und damit verhältnismäßig kostengünstig aus der Schmelze gezogen werden. Jedoch ist die Conturax®-Technologie nicht für Quarzglas einsetzbar, so dass hierfür auf andere Verfahren zurückgegriffen werden muss.

Ein Glasrohr kann auch durch entsprechende Nachbearbeitung mit entsprechenden Konturen, insbesondere Einbuchtungen, versehen werden. Beispielsweise können Konturen mittels Heißprägen und/oder Walzen in die Glasoberfläche eingebracht werden, bevorzugt ist das Heißprägen.

Die Wandstärke des Glasrohrs kann erfindungsgemäß zunächst beliebig eingestellt werden. Beschränkungen liegen nur im Hinblick auf den geplanten Einsatzzweck, die gewünschte Form und Größe sowie die gewünschten mechanischen Stabilitätsanforderungen vor. Beispielsweise liegen im Haushaltsbereich Außenanschlussdrücke von 4 bis 6 bar vor, die aber im weiteren Verlauf, z.B. bei Auslauf eines Wasserhahns, deutlich abfallen können, z.B. auf <1 bar. In der großtechnischen Wasseraufbereitung liegen die Drücke oft wesentlich höher, so dass das Glasrohr - je nach Einsatzzweck und Einsatzort - für bestimmte Drücke ausgelegt sein sollte. Dies stellt jedoch Wissen des Fachmanns dar, der ohne weiteres die geeignete Wandstärke für ein Glasrohr für das Anwendungsgebiet auswählen kann. Dem Fachmann aus dem Stand der Technik ist ebenfalls bekannt, wie derartige Glasrohre hergestellt werden können.

Das verwendbare UV-transparente Glas ist im Rahmen der vorliegenden Erfindung ebenfalls nicht besonders beschränkt. Es kann jedes dem Fachmann bekannte UV-transparente Glas zum Einsatz kommen. Erfindungsgemäß bevorzugte UV-transparente Gläser sind beispielsweise Quarzgläser, Silicatgläser, besonders bevorzugt Borosilcatgläser oder Natrium-Kalium-Barium-Silicatgläser, ganz besonders bevorzugt Quarzgläser und Borosilicatgläser.

Bei den erfindungsgemäß verwendeten Gläsern ist neben der erwünscht hohen UV-Durchlässigkeit darauf zu achten, dass diese gegenüber dem zu entkeimenden Medium eine ausreichende Stabilität aufweisen. Wenn beispielsweise Wasser entkeimt werden soll, wird vorzugsweise ein hydrolytisch ausreichend stabiles Glas eingesetzt. Nach DIN ISO 719 werden Gläser in 5 Wasserbeständigkeitsklassen eingeteilt. Wenn Wasser entkeimt werden soll, wird daher bevorzugt ein UV-transparentes Glas eingesetzt, das je nach gewählter Zusammensetzung, eine hydrolytische Beständigkeit der Klasse 1 bis 3 gemäß ISO 719 (auch bezeichnet als Wasserbeständigkeitsklasse oder WBK), besonders bevorzugt eine hydrolytische Beständigkeit der Klasse 1 gemäß ISO 719 aufweist.

Besonders bevorzugt eingesetzte UV-transparente Gläser weisen eine der nachfolgenden Glaszusammensetzungen (in Gew.-% auf Oxidbasis) auf:
Glaszusammensetzung 1:

| | | |
|---|---|---|
| SiO₂ | 75 - 85 | Gew.-% |
| B₂O₃ | 8 - 15 | Gew.-% |
| Al₂O₃ | 0,5 - 4 | Gew.-% |
| Na₂O | 1 - 6 | Gew.-% |
| K₂O | 0,1 - 2 | Gew.-% |
| ZrO₂ | < 0,005 | Gew.-% |

mit einem Gehalt an Fe₂O₃ < 100 ppm, bevorzugt < 10 ppm,
einem Gehalt an TiO₂ < 100 ppm, bevorzugt < 10 ppm
und einem Gehalt an Läutermittel;
oder
Glaszusammensetzung 2:

| | | |
|---|---|---|
| SiO₂ | 65 - 75 | Gew.-% |
| B₂O₃ | 15 - 22 | Gew.-% |
| Al₂O₃ | 4,5 - 7 | Gew.-% |
| Na₂O | 1,5 - 4 | Gew.-% |
| K₂O | 0,5 - 3 | Gew.-% |
| Li₂O | 0,1 - 1,5 | Gew.-% |
| BaO | 0,5 - 4 | Gew.-% |
| CaO | 0,1 - 2 | Gew.-% |
| MgO | <0,01 | Gew.-% |

mit einem Gehalt an Fe₂O₃ < 100 ppm, bevorzugt < 10 ppm,
einem Gehalt an TiO₂ < 100 ppm, bevorzugt < 10 ppm
und einem Gehalt an Läutermittel;
oder
Glaszusammensetzung 3:

| | | |
|---|---|---|
| SiO₂ | 65 - 78 | Gew.-% |
| B₂O₃ | 0,5 - 4 | Gew.-% |
| Al₂O₃ | 0,5 - 4 | Gew.-% |
| Na₂O | 5 - 10 | Gew.-% |
| K₂O | 8 - 14 | Gew.-% |
| BaO | 5 - 8 | Gew.-% |

mit einem Gehalt an Fe₂O₃ < 100 ppm, bevorzugt < 10 ppm,
einem Gehalt an Ti0₂ < 100 ppm, bevorzugt < 10 ppm,
einem Gehalt an Ca0 < 100 ppm, bevorzugt < 10 ppm,
einem Gehalt an MgO < 100 ppm, bevorzugt < 10 ppm
und einem Gehalt an Läutermittel.

Glas 1 ist beispielsweise für die Entkeimung von Wasser besonders bevorzugt, da es eine hydrolytische Beständigkeit der Klasse 1 aufweist. Die Gläser 2 und 3 werden besonders bevorzugt für die Entkeimung von Gasen verwendet.

Auch das zu entkeimende Medium ist erfindungsgemäß nicht besonders beschränkt. Es kann jede Flüssigkeit oder jedes Gas oder auch eine Mischung von mehreren Flüssigkeiten oder Gasen in der erfindungsgemäßen Vorrichtung behandelt werden. Ein bevorzugtes Medium ist Wasser. Wenn besonders aggressive Gase oder Flüssigkeiten entkeimt werden sollen, kann eine entsprechende Auswahl aus geeigneten Glaszusammensetzungen getroffen werden.

Gegenstand der Erfindung ist auch die Verwendung der erfindungsgemäßen Vorrichtung zur Entkeimung von Flüssigkeiten und/oder Gasen in ruhendem oder strömendem Zustand, insbesondere zur Trinkwasseraufbereitung und - entkeimung, Entkeimung von Reinstwasser, Abwasser, Flüssigkeiten aus dem pharmazeutischen und Lebensmittel-Bereich, zur Entkeimung von Gasen, wie Luft oder Industriegasen und dergleichen sowie bei der Reinstwassererzeugung.

Die Vorteile der Erfindung sind außerordentlich vielschichtig:
So stellt die Erfindung erstmals ein UV-transparentes Rohr, versehen mit mindestens einer Einbuchtung, in Verbindung mit mindestens einer UV-Lichtquelle zur Verfügung.

Nach einer besonders bevorzugten Ausführungsform der Erfindung sind die UV-Lichtquelle(n) in den ein oder mehreren Einbuchtung derart angeordnet, dass sich sämtliche UV-Lichtquellen zumindest teilweise, besonders bevorzugt vollständig, innerhalb der Rohraußenwand (bei nicht rundem Rohrquerschnitt), bevorzugt innerhalb des Rohraußenradius (bei rundem Rohrquerschnitt), des UV-transparenten Glasrohrs befinden, wobei man die Rohraußenwand bzw. den Rohraußenradius so auffasst, als ob die Einbuchtungen nicht vorhanden sind.

Noch bevorzugter sind die Einbuchtungen innerhalb der Rohrinnenwand (bei nicht rundem Rohrquerschnitt), bevorzugt innerhalb des Rohrinnenradius (bei rundem Rohrquerschnitt), des UV-transparenten Glasrohrs vorgesehen, wobei man die Rohrinnenwand bzw. den Rohinnenradius so auffasst, als ob die Einbuchtungen nicht vorhanden sind. Besonders bevorzugt sind erfindungsgemäß Anordnungen, bei denen sich die UV-Lichtquellen innerhalb der Rohrinnenwand bzw. innerhalb des Rohrinnenradius befinden, wie oben definiert, so dass eine besonders günstige Strahlungsverteilung resultiert, bei der nur noch ein geringer UV-Strahlungsanteil nicht auf direktem Wege durch das Glasrohr in den Rohrinnenraum gelangt.

Im Gegensatz zu Aussparungen - wie diese regelmäßig im Stand der Technik vorgesehen werden - bei denen üblicherweise eine größere Wandstärke des Rohrmaterials vorgesehen werden muss, um die UV-Lichtquellen unterzubringen, können bei den erfindungsgemäß vorgesehenen Einbuchtungen sehr viel geringere Wandstärken zum Einsatz kommen, so dass das Licht nur kurze Wege durch das Glas zurücklegt, und nicht bereits im Rohrmaterial zu hohen Anteilen absorbiert wird

Gegebenenfalls können zusätzlich zu den erfindungsgemäßen Einbuchtungen ein oder mehrere Aussparungen vorliegen, die bevorzugt keine UV-Lichtquellen aufweisen, sondern andere Funktionen haben. Beispielsweise kann eine Aussparung einen Sensor enthalten.

Nach einer weiteren bevorzugten Ausführungsform können auch nur Einbuchtungen im Glasrohr vorgesehen sein, Aussparungen liegen dann nicht vor.

Die erfindungsgemäße Geometrie bewirkt, dass die UV-Lichtquellen näher beim zu entkeimenden Medium vorliegen, d.h. in Richtung zum Innenraum des Rohrs versetzt sind. Es resultiert ein hoher Anteil an UV-Strahlung, der direkt in das Medium geführt wird, ohne vorher reflektiert zu werden. Durch die erfindungsgemäß bereitgestellte Geometrie wird weiterhin eine homogenere Lichtverteilung im Rohrinnenraum erzielt, wodurch die Entkeimungsleistung erhöht wird. Es resultiert somit eine höhere Gesamteffizienz des Systems.

Durch die erfindungsgemäße Anordnung wird zudem eine höhere Kompaktheit des Systems zur Verfügung gestellt.

Von großem Vorteil ist auch, dass die erfindungsgemäße Vorrichtung ein in sich geschlossenes System darstellt, das ohne Einwirkung von außen funktioniert. Während das zu entkeimende Medium, beispielsweise Wasser, durch das UV-transparente Glasrohr strömt, wird bestrahlt, ohne dass dem Medium irgendwelche Zusätze zugegeben werden müssten. Die UV-Lichtquellen werden gegebenenfalls sogar vom Medium umströmt, ohne in direkten Kontakt mit dem Medium zu kommen. Es wird daher größte Nähe zum Medium erreicht, aber gleichzeitig sind die UV-Lichtquellen durch eine Glasummantelung vor dem Medium geschützt.

Aufgrund der besonderen Anordnung der erfindungsgemäßen Vorrichtung kann das Glasrohr zusammen mit den zumindest teilweise in der Glaswand angeordneten UV-Lichtquellen und gegebenenfalls einem oder mehreren Reflektoren beispielsweise in einem kompakten Gehäuse untergebracht werden. Die Vorrichtung kann problemlos in größeren Einheiten, beispielsweise mit fließendem Medium, wie einem Rohrsystem, oder auch mit ruhendem Medium, wie einem Tank oder dergleichen, eingesetzt werden. Die Vorrichtung kann stationär, fest eingebaut, als Teil eines größeren Systems oder flexibel handhabbar als Handgerät zum Einsatz kommen. Die eigentliche Entkeimungsvorrichtung ist hierbei aus einem UV-transparenten Glasrohr mit darin vorzugsweise parallel zur Rohrachse verlaufenden Einbuchtungen angeordneten UV-Lichtquellen und vorzugsweise einem oder mehreren Reflektoren aufgebaut.

Ferner ist die Herstellung speziell ausgeformter UV-transparenter Glasrohre, die in der erfindungsgemäßen Vorrichtung Verwendung finden, in einfacher Weise möglich.

Die Besonderheit der Erfindung ist daher die Nutzung der speziellen Glasrohrform mit Einbuchtungen. Durch die erfindungsgemäß mögliche besondere Bestrahlungsgeometrie wird eine sehr hohe Entkeimungsleistung erzielt. Die erfindungsgemäße Vorrichtung erzielt damit einen möglichst hohen Wirkungsgrad bei relativ geringem Kostenaufwand bei der Herstellung.

Die erfindungsgemäße Vorrichtung ist auch für sehr spezielle Anforderungen geeignet. Beispielsweise für die Herstellung von hochreinem Wasser, das insbesondere im Bereich der Pharma-, Kosmetik- und Halbleiterindustrie benötigt wird.

Die Vorrichtung zeigt Ihre Vorteile insbesondere bei kleineren Systemen mit hoher Kompaktheit, beispielsweise mit Drücken im Haushaltsbereich. Durch die erfindungsgemäße Vorrichtung wird der Wirkungsgrad gegenüber bekannten Anwendungen deutlich erhöht.

Die vorliegende Erfindung wird nachfolgend anhand der beiliegenden Zeichnungen detailliert beschrieben, welche die vorliegende Erfindung nicht beschränken sollen. Es zeigen:
- Figur 1a, b und c: jeweils schematische Querschnittsansichten von beispielhaften Ausführungsformen aus dem Stand der Technik gemäß der DE 10 2010 005 893 A1 (dort die Figuren 6b, 6c und 6d)
- Figur 2: eine schematische Querschnittsansicht einer beispielhaften erfindungsgemäßen Ausführungsform eines runden UV-transparenten Glasrohrs mit 3 UV-Lichtquellen, die sich jeweils in einer Einbuchtung befinden, und einem Reflektor, wobei um das Glasrohr und die UV-Lichtquellen ein Reflektor vorgesehen ist;
- Figur 3: eine schematische Querschnittsansicht einer weiteren beispielhaften erfindungsgemäßen Ausführungsform eines runden UV-transparenten Glasrohrs mit 3 UV-Lichtquellen, wobei das Rohr außen einen Reflektor aufweist und zusätzlich jeder UV-Lichtquelle ein Reflektor zugeordnet ist;
- Figur 4: eine schematische Querschnittsansicht einer weiteren beispielhaften erfindungsgemäßen Ausführungsform eines runden UV-transparenten Glasrohrs mit 3 UV-Lichtquellen, wobei das Rohr innen einen Reflektor aufweist und zusätzlich jeder UV-Lichtquelle außen ein Reflektor zugeordnet ist;
- Figur 5a: eine dreidimensionale schematische Ansicht einer beispielhaften erfindungsgemäßen Ausführungsform einer Vorrichtung der Erfindung;
- Figur 5b: eine dreidimensionale schematische Ansicht einer beispielhaften erfindungsgemäßen Ausführungsform eines UV-transparenten Glasrohrs mit mehreren UV-Lichtquellen;
- Figur 6: eine graphische Darstellung, bei der die Transmission gegen die Wellenlänge (Transmissionsverlauf) für 3 bevorzugt verwendete UV-transparente Gläser aufgetragen ist.

Die verschiedenen in der Zeichnung dargestellten Elemente sind nur repräsentativ und nicht notwendigerweise im Maßstab gezeichnet. Bestimmte Abschnitte hiervon können übertrieben sein, während andere minimiert sein können. Die Zeichnung soll eine beispielhafte Ausführungsform der Offenbarung veranschaulichen, die vom Fachmann im Stand der Technik verstanden und geeigneterweise ausgeführt werden kann. In den Figuren werden gleiche Komponenten und Elemente mit gleichen Bezugszeichen und Symbolen bezeichnet.

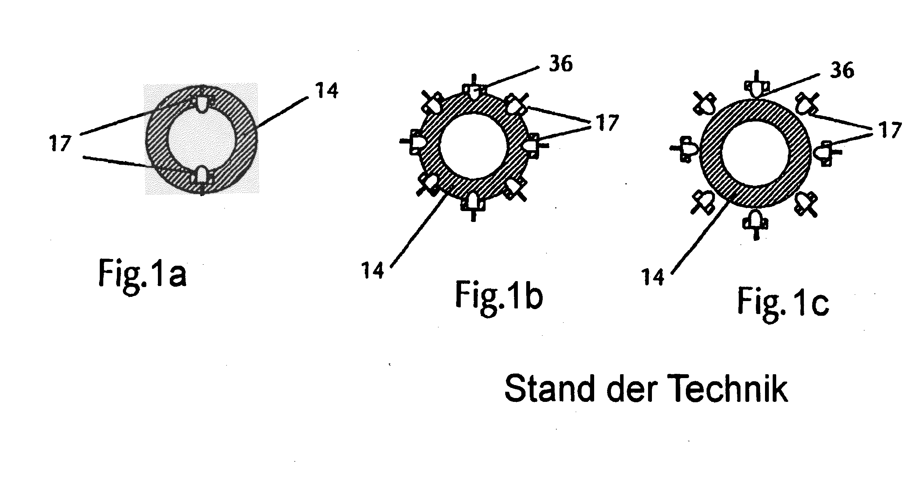

Figuren 1a, 1b und 1c zeigen jeweils schematische Querschnittsansichten von beispielhaften Ausführungsformen aus dem Stand der Technik gemäß der DE 10 2010 005 893 A1 (dort die Figuren 6b, 6c und 6d).

In Figur 1a ist eine UV-Bestrahlungseinheiten dargestellt, die eine UV-Bestrahlungseinrichtung 17 in die Wandung eines Leitungssystems 14 integriert zeigt. Die UV-Bestrahlungseinrichtungen 17 stehen mit ihren Spitzenbereichen jeweils in das Leitungsinnere vor. Die Lichtquellen befinden sich daher in direktem Kontakt mit dem im Innenraum fließenden Medium und müssen gegen dieses beständig und abgedichtet sein. Ein einfacher Austausch ist nicht mehr ohne weiteres möglich. Hierfür muss das System abgeschaltet werden.

In Figur 1b sind eine Vielzahl von UV-Bestrahlungseinrichtungen 17 in Form von UV-LEDs 36 dargestellt, die in die Wandung des Leitungssystems 14 integriert sind. Die UV-LEDs 36 befinden sich demnach in Aussparungen der Rohrwand. Die gezeigten Aussparungen aus dem Stand der Technik zeigen, dass regelmäßig eine größere Wandstärke des Rohrmaterials vorliegt, um die UV-Lichtquellen unterzubringen. Hieraus resultiert eine Verlängerung des Wegs, den das UV-Lichts durch das Rohr zum Medium zurücklegen muss, wodurch Verluste auftreten. Zudem wird ein größerer Teil des von der UV-Lichtquelle seitlich abgestrahlten Lichts das Rohr nach außen verlassen oder in der Rohrwand geführt werden und ist daher nicht für die Entkeimung nutzbar. Diese Geometrie eignet sich daher nur schlecht für ungerichtete Lichtquellen.

In Figur 1c sind wieder eine Vielzahl von UV-LEDs 36 außerhalb des Leitungssystems 14 angeordnet, so dass die gesamte Wandstärke des Rohrs zwischen den UV-Lichtquellen und dem zu entkeimenden Medium vorhanden ist. Verluste an UV-Licht sind daher aufgrund von Restabsorption zwangsläufig. Analog zu Fig. 1b eignet sich auch diese Anordnung nur schlecht für ungerichtete Lichtquellen. Ein weiterer Nachteil dieser Anordnung ist der große Bauraum, wobei eine Vielzahl von UV-Lichtquellen um das Rohr herum angeordnet werden muss, um eine ausreichende Entkeimung zu gewährleisten. Derartige Anlagen sind nicht kompakt, sondern benötigen viel Platz.

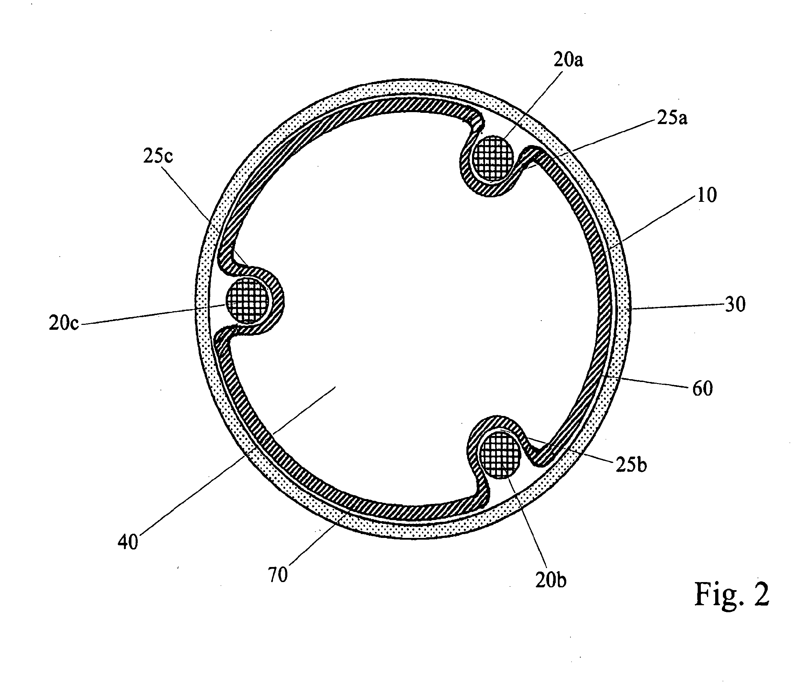

Figur 2 zeigt eine schematische Querschnittsansicht einer beispielhaften erfindungsgemäßen Ausführungsform eines Rohrs 10 mit 3 UV-Lichtquellen 20a, 20b und 20c und einem Reflektor 30. Die in Figur 2 dargestellte Ausführungsform weist einen rohrförmigen Innenraum 40 auf, durch den senkrecht zur Zeichenebene eine Flüssigkeit und/oder ein Gas strömt. Das Rohr 10 ist aus einem UV-transparenten Glas aufgebaut. Das Glas kann beliebig ausgewählt werden, sofern es für den Einsatzzweck geeignet ist. Die Rohrwand weist 3 Einbuchtungen 25a, 25b und 25c auf.

Im Gegensatz zu den Ausführungsformen mit Aussparungen aus dem Stand der Technik gemäß der Figuren 1a, 1b und 1c sind in Figur 2 erfindungsgemäß Einbuchtungen vorgesehen. Hierbei liegt in der Regel ein Unterschied hinsichtlich der Wandstärke des Glasrohrs 10 vor, da beim Vorsehen von Aussparungen zweckmäßigerweise eine größere Wandstärke vorgesehen wird, um die UV-Lichtquellen unterzubringen, als beim Vorsehen von Einbuchtungen.

Um eine Einbuchtung zu erhalten, ist sowohl die Rohraußenwand 60 als auch die Rohrinnenwand 70 gleichzeitig an der selben Stelle nach innen gestülpt, so dass die Einbuchtungen 25a, 25b und 25c in den Innenraum 40 hinein reichen. Die Einbuchtungen 25a, 25b und 25c bilden jeweils Hohlräume oder Vertiefungen aus, in denen jeweils UV-Lichtquellen 20a, 20b und 20c angeordnet sind, die vorzugsweise parallel zur Strömungsrichtung des zu entkeimenden Mediums angeordnet sind. Die UV-Lichtquellen 20a, 20b und 20c werden im vorliegenden Fall vollständig von den dargestellten Einbuchtungen 25a, 25b und 25c aufgenommen und befinden sich praktisch vollständig innerhalb der Rohrinnenwand 70, im gezeigten Fall des Rohrinnenradius, wenn man das Rohr 10 so ausführen würde, dass keine Einbuchtungen vorhanden sind. Im gezeigten Beispielfall stimmt die Anzahl der UV-Lichtquellen 20a, 20b und 20c mit der Anzahl der Einbuchtungen 25a, 25b und 25 c überein, so dass in jeder Einbuchtung 25a, 25b und 25c jeweils eine UV-Lichtquelle 20a, 20b und 20c vorhanden ist. Beispielsweise könnten auch zusätzliche Einbuchtungen vorliegen oder in einer Einbuchtung könnte mehr als eine UV-Lichtquelle, beispielsweise ein Bündel von UV-Lichtquellen, vorliegen.

Weiterhin sind in Fig. 2 die gezeigten Einbuchtungen 25a, 25b und 25c an die Form der UV-Lichtquellen 20a, 20b und 20 c angepasst, so dass diese vollständig in den gebildeten Hohlraum aufgenommen werden und nicht mehr nach außen überstehen.

Die in Fig. 2 gezeigten 3 UV-Lichtquellen 20a, 20b und 20c sind im runden Glasrohr 10 symmetrisch angeordnet, d.h. diese spannen ein gleichseitiges Dreieck auf, mit den UV-Lichtquellen 20a, 20b und 20c jeweils an den Ecken des Dreiecks. Das UV-Licht muss aufgrund der nach innen reichenden Einbuchtungen 25a, 25b und 25c einen kürzeren Weg durch die Rohrwand nehmen, um zum Innenraum 40 zu gelangen, wo sich das zu entkeimende Medium befindet. Dadurch kann ein großer Anteil des UV-Lichts auf direktem Weg durch die Rohrwand in den Innenraum 40 gelangen, wo sich das zu entkeimende Medium befindet. Diese Geometrie ist vorteilhaft, um eine möglichst homogene Strahlungsverteilung mit hoher Strahlungsdichte zu erhalten, wobei ein üblicherweise zwischen den einzelnen UV-Lichtquellen auftretendes Strahlungsintensitätsminimum weitestgehend vermieden werden kann.

Selbstverständlich sind auch andere Rohrquerschnitte und Geometrien mit einer anderen Zahl von Einbuchtungen und eine andere Zahl an UV-Lichtquellen als die gezeigten möglich.

Neben den Einbuchtungen 25a, 25b und 25c können in Fig.2 auch ein oder mehrere Aussparungen (nicht gezeigt) im Glasrohr 10 vorgesehen sein. Diese enthalten vorzugsweise keine UV-Lichtquelle, sondern beispielsweise einen Sensor.

Weiterhin ist in Fig. 2 ein Reflektor 30 vorgesehen, um das nach außen abgestrahlte UV-Licht wieder in das Rohr 10 hinein zu reflektieren. Die Art, Form Größe und der Aufbau des Reflektors 30 sind erfindungsgemäß nicht weiter beschränkt. Ein Reflektor 30 kann jede Art einer beschichteten Oberfläche zur Reflektion von Licht darstellen, wie eine Reflexfolie, ein Spiegel und dergleichen. Im gezeigten Beispielfall ist der Reflektor 30 um die gesamte Anordnung herum liegend angeordnet. Der Reflektor 30 hat eine runde Form und liegt an der Rohraußenwand 60 an. Da die drei Einbuchtungen 25a, 25b und 25c derart ausgestaltet sind, dass die darin vorliegenden drei UV-Lichtquellen 20a, 20b und 20 c vollständig von den Einbuchtungen 25a, 25b und 25c aufgenommen sind und damit in Richtung des Innenraums 40 des Rohrs 10 versetzt untergebracht werden und nicht mehr nach außen überstehen, kann der Reflektor 30 direkt auf die Rohraußenwand 60 aufgebracht werden. Durch den gezeigten Aufbau wird eine besonders einfache Reflektorgeometrie möglich, der Reflektor 30 liegt direkt am Rohr 10 an und wird durch dieses gehalten und stabilisiert. Der Reflektor kann auch direkt am Rohr 10 befestigt oder auf dieses aufgebracht sein. In der vorliegenden Ausführungsform der Erfindung wird ein besonders einfacher Aufbau der erfindungsgemäßen Vorrichtung erzielt, da die Vorrichtung vollständig von einem Rohr umschlossen werden kann, das gleichzeitig als Reflektor dient und am Rohr 10 unmittelbar anliegt. Der Reflektor 30 kann in besonders vorteilhafter Weise auch eine UV-reflektierende Beschichtung darstellen, die unmittelbar auf das Rohr 10 aufgebracht, beispielsweise aufgedampft wird.

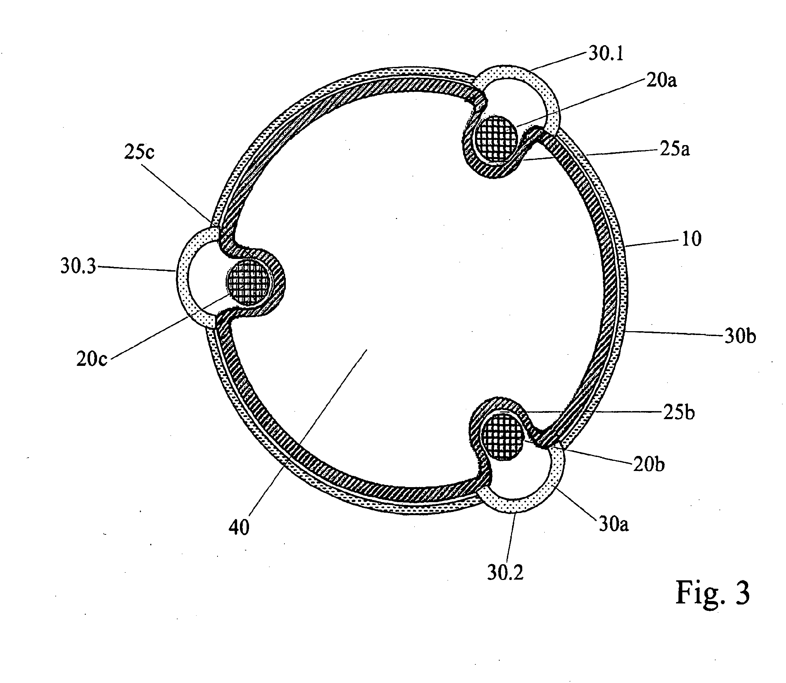

Figur 3 zeigt eine schematische Querschnittsansicht einer weiteren beispielhaften erfindungsgemäßen Ausführungsform eines Rohrs 10 mit 3 UV-Lichtquellen 20a, 20b und 20c und Reflektoren 30a und 30b. Die erfindungsgemäße Ausführungsform ist ähnlich zu der in Fig. 2 gezeigten, aber zusätzlich ist jeder UV-Lichtquelle 20a, 20b und 20c jeweils ein Reflektor 30a (30.1, 30.2 und 30.3) zugeordnet.

Die einzelnen den jeweiligen UV-Lichtquellen 20a, 20b und 20c zugeordneten Reflektoren 30a (30.1, 30.2 und 30.3) sind in Form, Größe und Gestalt frei wählbar. Im gezeigten Beispielfall sind die Reflektoren 30.1, 30.2 und 30.3 in Form von Kugelausschnitten dargestellt. Die gezeigten 3 Reflektoren 30.1, 30.2 und 30.3 sind alle gleich groß und mit gleicher Form dargestellt. Dies ist jedoch nicht in jedem Fall erforderlich. Es sind auch andere Reflektorgrößen und -designs möglich, die bei jeder UV-Lichtquelle 20a, 20b und 20c anders ausgestaltet sein können.

Neben den um die 3 UV-Lichtquellen 20a, 20b und 20c angeordneten 3 Reflektoren 30a (30.1, 30.2 und 30.3) befindet sich ein weiterer Reflektor 30b um das Rohr 10 selbst angeordnet. Im gezeigten Aufbau befindet sich ein Reflektor 30b direkt um das Rohr 10 angeordnet und wird durch dieses gehalten und stabilisiert. Der Reflektor kann auch direkt am Rohr 10 befestigt oder in Form einer UV-reflektierenden Beschichtung auf dieses aufgebracht werden.

Das UV-Licht muss aufgrund der in den Einbuchtungen 25a, 25b und 25c vorliegenden UV-Lichtquellen 20a, 20b und 20c, die so angeordnet sind, dass sie sich innerhalb der (ohne Einbuchtungen vorliegenden) Rohrinnenwand 60 befinden, einen kürzeren Weg zum Innenraum 40 nehmen, wo sich das zu entkeimende Medium befindet. Der Anteil des UV-Lichts der zunächst über einen Reflektor reflektiert wird, bevor er in den Innenraum gelangt, wird deutlich reduziert, eine verlustarme Einkopplung des UV-Lichts resultiert, wodurch eine bessere Ausnutzung der UV-Strahlung erreicht wird.

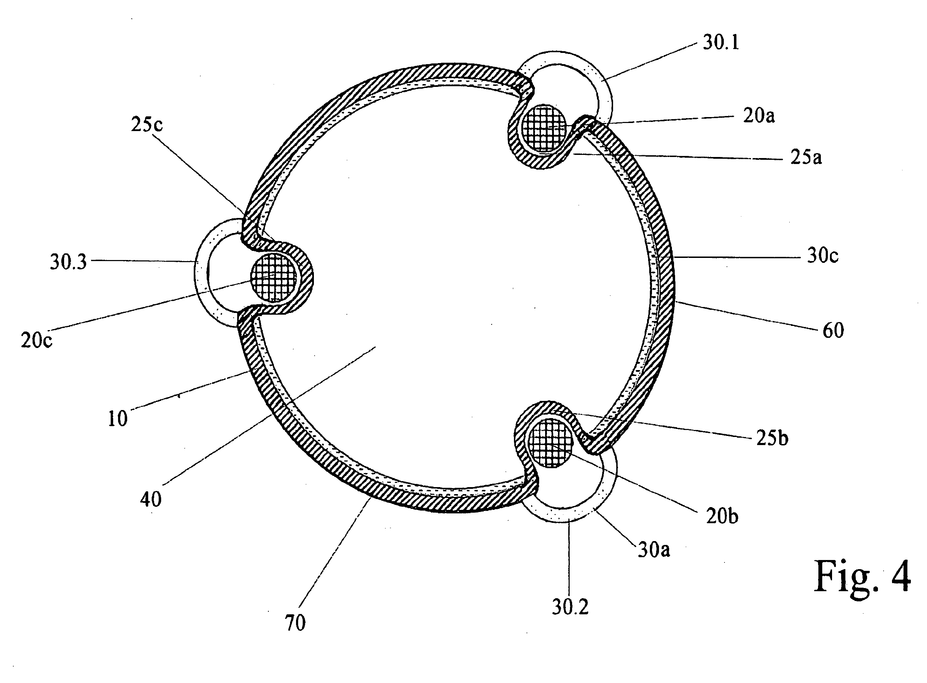

In Figur 4, die ähnlich zu Figur 3 ist, wird eine schematische Querschnittsansicht einer weiteren beispielhaften erfindungsgemäßen Ausführungsform eines runden UV-transparenten Glasrohrs mit 3 UV-Lichtquellen gezeigt, wobei das Rohr innen einen Reflektor aufweist und zusätzlich jeder UV-Lichtquelle ein Reflektor zugeordnet ist. Der Innenreflektor ist im Bereich der Einbuchtungen 25a, 25b und 25c selbstverständlich ausgespart. Die obigen Ausführungen zu Figur 3 gelten entsprechend.

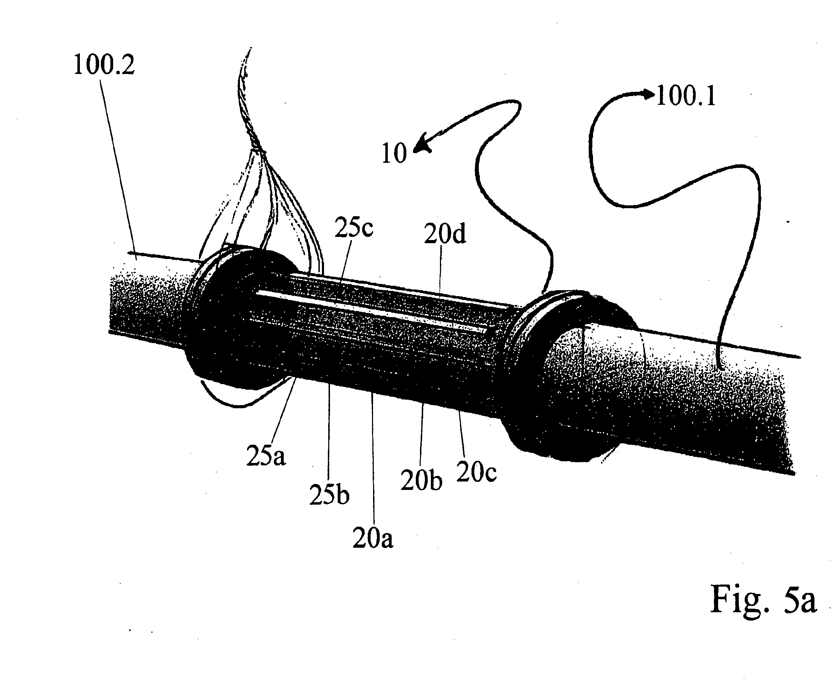

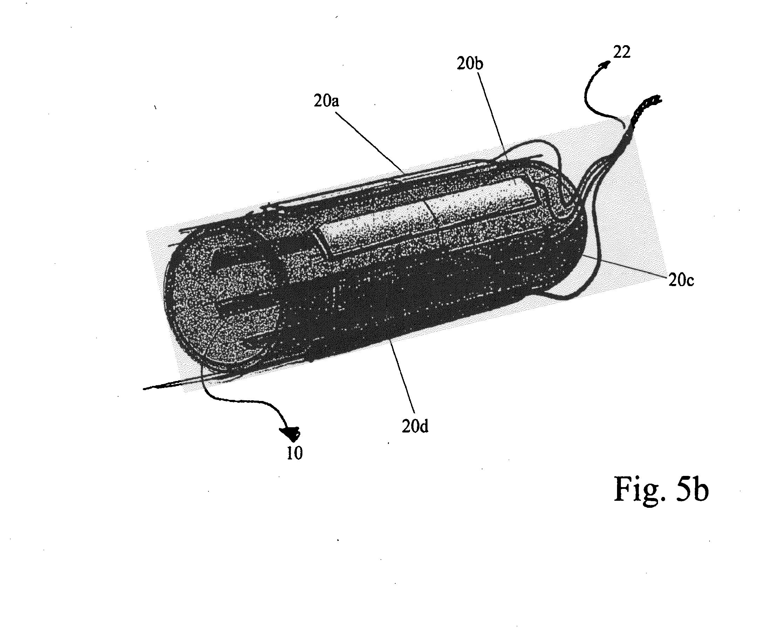

In Figur 5a ist eine dreidimensionale schematische Ansicht einer beispielhaften Vorrichtung gemäß einer erfindungsgemäßen Ausführungsform gezeigt. Das Rohr 10 ist in einem Rohrsystem eingebaut, beispielsweise eingebaut zwischen den Metallrohren 100.1 und 100.2. Das Rohr 10 ist aus UV-transparentem Glas aufgebaut und weist Einbuchtungen 25a, 25b, 25c.... auf, in denen sich die UV-Lichtquellen 20a, 20b, 20c... befinden. In Figur 5b ist eine dreidimensionale schematische Ansicht des Rohrs 10 von Fig. 5a mit mehreren UV-Lichtquellen 20a, 20b, 20c... dargestellt, die über die Anschlüsse 22 mit einer Stromquelle verbunden sind.

Das verwendete UV-transparente Glas ist nicht besonders beschränkt. Für besonders bevorzugt eingesetzte UV-transparente Gläser ist in Figur 6 der Transmissionsverlauf in einer graphischen Darstellung gezeigt. Hierbei wurde die Transmission (in %) gegen die Wellenlänge (in nm) für einige bevorzugt verwendete UV-transparente Gläser aufgetragen. Fig. 6 zeigt die UV-Transmission für Glas 1, Glas 2 und Glas 3.

Die Figuren 1 bis 6 verdeutlichen nur beispielhaft mögliche Ausgestaltungen. Diese sind nicht beschränkend zu verstehen, sondern stellen lediglich Beispiele möglicher Ausführungsformen dar. Andere Möglichkeiten zur Ausführung sind denkbar.

### Bezugszeichenliste

- 10: Glasrohr
- 14: Leitungssystem aus dem Stand der Technik
- 17: UV-Bestrahlungseinrichtung aus dem Stand der Technik
- 20a, 20b, 20c...: UV-Lichtquelle
- 25a, 25b, 25c...: Einbuchtung
- 22: Anschlüsse
- 30, 30a, 30b, 30.1, 30.2, 30.3...: Reflektor
- 36: UV-LEDs aus dem Stand der Technik
- 40: Rohrinnenraum
- 60: Rohraußenwand
- 70: Rohrinnenwand
- 100.1, 100.2: Metallrohr

## Patentansprüche

1. Vorrichtung zur Entkeimung von Gasen oder Flüssigkeiten, umfassend ein Rohr (10) aus UV-transparentem Glas, das einen hohlen Innenraum (40) und eine Rohrwand mit einer Rohrinnenwand (70) und einer Rohraußenwand (60) aufweist, sowie mindestens eine UV-Lichtquelle (20a, 20b, 20c...), wobei das UV-transparente Glasrohr (10) an mindestens einer Stelle
eine Einbuchtung (25a, 25b, 25c...) in den Innenraum (40)
aufweist und
in der mindestens einen Einbuchtung (25a, 25b, 25c...) die UV-Lichtquelle, (20a, 20b, 20c...) angeordnet ist,
**dadurch gekennzeichnet, dass**
die Einbuchtung eine Einstülpung der Glaswand des Glasrohres vorbestimmter Größe darstellt, wobei die Rohrinnenwand und die Rohraußenwand gleichzeitig an derselben Stelle nach Innen gestülpt sind und zusammen einen zur Rohraußenseite offenen Hohlraum ausbilden, in den die mindestens eine UV-Lichtquelle aufgenommen ist.

2. Vorrichtung nach Anspruch 1, **dadurch gekennzeichnet, dass** die mindestens eine UV-Lichtquelle (20a, 20b, 20c...) in der mindestens einen Einbuchtung (25a, 25b, 25c...) derart angeordnet ist, dass sich die mindestens eine UV-Lichtquelle (20a, 20b, 20c...) zumindest teilweise innerhalb der Rohrinnenwand (60) des UV-transparenten Glasrohrs (10) befindet, wenn die Rohrinnenwand (60) ohne Einbuchtungen vorliegen würde.

3. Vorrichtung nach mindestens einem der vorangehenden Ansprüche 1 oder 2, **dadurch gekennzeichnet, dass** die Anzahl der Einbuchtungen (25a, 25b, 25c...) mit der Anzahl der UV-Lichtquellen (20a, 20b, 20c...) übereinstimmt und jeweils nur eine UV-Lichtquelle (20a, 20b, 20c...) in einer Einbuchtung (25a, 25b, 25c...) vorgesehen ist.

4. Vorrichtung nach mindestens einem der vorangehenden Ansprüche 1 bis 3, **dadurch gekennzeichnet, dass** die Anzahl der UV-Lichtquellen (20a, 20b, 20c...), die im UV-transparenten Glasrohr (10) vorgesehen sind, ausgewählt ist aus 1 bis 4 UV-Lichtquellen (20a, 20b, 20c...).

5. Vorrichtung nach mindestens einem der vorangehenden Ansprüche 1 bis 4, **dadurch gekennzeichnet, dass** das UV-transparente Glas ausgewählt ist aus Quarzglas, Silicatglas, bevorzugt Borosilicatglas, Natrium-Kalium-Barium-Silicatglas.

6. Vorrichtung nach mindestens einem der vorangehenden Ansprüche, **dadurch gekennzeichnet, dass**
- ein Reflektor (30, 30b) um das Glasrohr (10) herum angeordnet ist und dieses vollständig einhüllt oder
- ein Reflektor (30, 30b) um das Glasrohr (10) herum angeordnet ist oder an der Rohraußenwand (60) angebracht ist, ausgenommen im Bereich der Einbuchtungen (25a, 25b, 25c...), wobei für jede UV-Lichtquelle (20a, 20b, 20c...) ein zusätzlicher Reflektor (30a, 30.1, 30.2, 30.3...) vorgesehen ist oder
- ein Reflektor (30, 30c) auf der Rohrinnenwand (70) angebracht ist, ausgenommen im Bereich der Einbuchtungen (25a, 25b, 25c...), wobei für jede UV-Lichtquelle (20a, 20b, 20c...) ein zusätzlicher Reflektor (30a, 30.1, 30.2, 30.3...) vorgesehen ist.

7. Vorrichtung nach Anspruch 6, **dadurch gekennzeichnet, dass** der Reflektor (30, 30b) auf die Rohraußenwand (60) oder Rohrinnenwand (70), in Form einer UV-reflektierenden Beschichtung aufgebracht ist, ausgenommen dem Bereich, wo die Einbuchtungen (25a, 25b, 25c...) vorliegen..

8. Vorrichtung nach mindestens einem der vorangehenden Ansprüche, **dadurch gekennzeichnet, dass** die mindestens eine UV-Lichtquelle (20a, 20b, 20c...) ausgewählt ist aus einer Mitteldruck-, Hochdruck- oder Niederdruck-UV-Lampe, CCL oder UV-LEDs.

9. Vorrichtung nach mindestens einem der vorangehenden Ansprüche, **dadurch gekennzeichnet, dass** die Einbuchtungen (25a, 25b, 25c...) lokal begrenzt im Glasrohr (10) vorgesehen sind.

10. Vorrichtung nach mindestens einem der vorangehenden Ansprüche, **dadurch gekennzeichnet, dass** die Form der Einbuchtungen (25a, 25b, 25c...) an die Form der verwendeten UV-Lichtquelle (20a, 20b, 20c...) angepasst ist.

11. Vorrichtung nach mindestens einem der vorangehenden Ansprüche, **dadurch gekennzeichnet, dass**
- bei Vorsehen von ein oder mehreren Einbuchtungen (25a, 25b, 25c...) die Wandstärke des Glasrohres (10) längs des Rohrumfangs konstant ist.

12. Vorrichtung nach mindestens einem der vorangehenden Ansprüche, **dadurch gekennzeichnet, dass** der Querschnitt des Rohrs (10) ausgewählt ist aus rund, oval, eckig, bevorzugt rund oder oval.

13. Vorrichtung nach mindestens einem der vorangehenden Ansprüche, **dadurch gekennzeichnet, dass** die UV-Lichtquellen (20a, 20b, 20c...) im Glasrohr (10) symmetrisch angeordnet sind.

14. Verwendung einer Vorrichtung nach einem der Ansprüche 1 bis 13 zur Entkeimung von Flüssigkeiten und/oder Gasen in ruhendem oder strömendem Zustand, insbesondere zur Trinkwasseraufbereitung und -entkeimung, Entkeimung von Reinstwasser, Abwasser, Flüssigkeiten aus dem pharmazeutischen und Lebensmittel-Bereich, zur Entkeimung von Gasen, wie Luft oder Industriegasen und dergleichen sowie bei der Reinstwassererzeugung.

## Claims

1. An apparatus for sterilizing gases or liquids, comprising a tube (10) made of UV-transparent glass, which has a hollow interior space (40) and a tube wall with an inner wall (70) and an outer wall (60), and at least one UV light source (20a, 20b, 20c ....), wherein the UV-transparent glass tube (10) comprises at least one depression (25a, 25b, 25c ....) into the interior space (40) at least at one place, and the UV light source (20a, 20b, 20c...) is arranged in the at least one depression (25a, 25b, 25c...), **characterized in that** the depression represents an indentation of the glass wall of the glass tube of a predetermined size, wherein the inner wall and the outer wall of the tube are simultaneously indented inwardly at the same point and jointly form a hollow space which is open towards the outside of the tube, in which at least one UV light source is accommodated.

2. An apparatus according to claim 1, **characterized in that** the at least one UV light source (20a, 20b, 20c ...) in the at least one depression (25a, 25b, 25c ...) is arranged in such a way that the at least one UV light source (20a, 20b, 20c ...) is situated at least partly within the inner wall (60) of the UV-transparent glass tube (10) if the inner wall (60) of the tube were present without the indentations.

3. An apparatus according to at least one of the preceding claims 1 or 2, **characterized in that** the number of the depressions (25a, 25b, 25c ...) corresponds to the number of the UV light sources (20a, 20b, 20c ...), and only one respective UV light source (20a, 20b, 20c ...) is provided in a depression (25a, 25b, 25c ...).

4. An apparatus according to at least one of the preceding claims 1 to 3, **characterized in that** the number of the UV light sources (20a, 20b, 20c ...) which are provided in the UV-transparent glass tube (10) is chosen from one to four UV light sources (20a, 20b, 20c ...).

5. An apparatus according to at least one of the preceding claims 1 to 4, **characterized in that** the UV-transparent glass is chosen from silica glass, silicate glass, preferably borosilicate glass, sodium-potassium-barium silicate glass.

6. An apparatus according to at least one of the preceding claims, **characterized in that**
- a reflector (30, 30b) is arranged around the glass tube (10) and completely encloses the same, or
- a reflector (30, 30b) is arranged around the glass tube (10) or is attached to an outside wall (60) of the tube, with the exception in the region of the depressions (25a, 25b, 25c ...), wherein an additional reflector (30a, 30.1, 30.2, 30.3...) is provided for each UV light source (20a, 20b, 20c ...), or
- a reflector (30, 30b) is arranged on the inner wall (70) of the tube, with the exception in the region of the depressions (25a, 25b, 25c ...), wherein an additional reflector (30a, 30.1, 30.2, 30.3...) is provided for each UV light source (20a, 20b, 20c ...).

7. An apparatus according to claim 6, **characterized in that** the reflector (30, 30b) is applied to the outer wall (60) or inner wall (70) of the tube in form of a UV-reflecting coating, with the exception of the region where the depressions (25a, 25b, 25c ...) are present.

8. An apparatus according to at least one of the preceding claims, **characterized in that** the at least one UV light source (20a, 20b, 20c ...) is chosen from a medium-pressure, high-pressure or low-pressure UV lamp, CCL or UV LED.

9. An apparatus according to at least one of the preceding claims, **characterized in that** the depressions (25a, 25b, 25c ...) are present in a locally limited manner in the glass tube (10).

10. An apparatus according to at least one of the preceding claims, **characterized in that** the shape of the depressions (25a, 25b, 25c ...) is adapted to the shape of the used UV light source (20a, 20b, 20c ...).

11. An apparatus according to at least one of the preceding claims, **characterized in that**
- the wall thickness of the glass tube (10) is constant along the circumference of the tube when one or several depressions (25a, 25b, 25c ...) are provided.

12. An apparatus according to at least one of the preceding claims, **characterized in that** the cross-section of the tube (10) is chosen from round, oval, angular, preferably round or oval.

13. An apparatus according to at least one of the preceding claims, **characterized in that** the UV light sources (20a, 20b, 20c ...) are arranged symmetrically in the glass tube (10).

14. The use of an apparatus according to one of the claims 1 to 13 for sterilising liquids and/or gases in the static or flowing state, especially for conditioning and sterilising drinking water, sterilising ultrapure water, wastewater, liquids from the pharmaceutical and foodstuff areas, for sterilising gases such as air or industrial gases and the like, and in the production of ultrapure water.

## Revendications

1. Dispositif pour la désinfection de gaz ou de liquides, comprenant un tube (10) en verre transparent aux UV, présentant un espace intérieur creux (40) et une paroi de tube avec une paroi intérieure de tube (70) et une paroi extérieure de tube (60), ainsi qu'au moins une source de lumière UV (20a, 20b, 20c...), dans lequel le tube en verre transparent aux UV (10) présente à au moins un endroit un creux (25a, 25b, 25c...) dans l'espace intérieur (40) et la source de lumière UV (20a, 20b, 20c...) est disposée dans l'au moins un creux (25a, 25b, 25c...), **caractérisé en ce que** le creux représente un évasement de la paroi en verre du tube en verre de taille prédéterminée, la paroi intérieure du tube et la paroi extérieure du tube étant évasées en même temps vers l'intérieur au même endroit et formant ensemble une cavité ouverte vers l'extérieur du tube, dans laquelle l'au moins une source de lumière UV est logée.

2. Dispositif selon la revendication 1, **caractérisé en ce que** l'au moins une source de lumière UV (20a, 20b, 20c...) est disposée dans l'au moins un creux (25a, 25b, 25c...) de telle manière que l'au moins une source de lumière UV (20a, 20b, 20c...) se trouve au moins partiellement à l'intérieur de la paroi intérieure de tube (60) du tube en verre transparent aux UV (10) si la paroi intérieure du tube (60) était dépourvue de creux.

3. Dispositif selon l'une au moins des revendications 1 ou 2, **caractérisé en ce que** le nombre de creux (25a, 25b, 25c...) coïncide avec le nombre de sources de lumière UV (20a, 20b, 20c...) et une seule source de lumière UV (20a, 20b, 20c...) est prévue respectivement dans un creux (25a, 25b, 25c...).

4. Dispositif selon l'une au moins des revendications 1 à 3, **caractérisé en ce que** le nombre de sources de lumière UV (20a, 20b, 20c...) prévu dans le tube en verre transparent aux UV (10) est choisi entre 1 et 4 sources de lumière UV (20a, 20b, 20c...)

5. Dispositif selon l'une au moins des revendications 1 à 4, **caractérisé en ce que** le verre transparent aux UV est choisi parmi le verre au quartz, le verre au silicate, de préférence le verre borosilicate, le verre de silicate de sodium et potassium.

6. Dispositif selon l'une au moins des revendications précédentes, **caractérisé en ce que**
- un réflecteur (30, 30b) est disposé autour du tube en verre (10) et enveloppe complètement celui-ci ou
- un réflecteur (30, 30b) est disposé autour du tube en verre (10) ou appliqué sur la paroi extérieure du tube (60), sauf dans la région des creux (25a, 25b, 25c...), un réflecteur supplémentaire (30a, 30.1, 30.2, 30.3...) étant prévu pour chaque source de lumière UV (20a, 20b, 20c...), ou
- un réflecteur (30, 30c) est appliqué sur la paroi intérieure du tube (70), sauf dans la région des creux (25a, 25b, 25c...), un réflecteur supplémentaire (30a, 30.1, 30.2, 30.3...) étant prévu pour chaque source de lumière UV (20a, 20b, 20c...).

7. Dispositif selon la revendication 6, **caractérisé en ce que** le réflecteur (30, 30b) est appliqué sur la paroi extérieure du tube (60) ou la paroi intérieure du tube (70) sous la forme d'un revêtement réfléchissant les UV, sauf dans la zone où se trouvent les creux (25a, 25b, 25c...).

8. Dispositif selon l'une au moins des revendications précédentes, **caractérisé en ce que** l'au moins une source de lumière UV (20a, 20b, 20c...) est choisie parmi une lampe à UV à moyenne pression, haute pression ou basse pression, une lampe CCFL ou une LED à UV.

9. Dispositif selon l'une au moins des revendications précédentes, **caractérisé en ce que** les creux (25a, 25b, 25c...) sont prévus dans le tube en verre (10) de façon localement circonscrite.

10. Dispositif selon l'une au moins des revendications précédentes, **caractérisé en ce que** la forme des creux (25a, 25b, 25c...) est adaptée à celle de la source de lumière UV (20a, 20b, 20c...) utilisée.

11. Dispositif selon l'une au moins des revendications précédentes, **caractérisé en ce que** lorsqu'un ou plusieurs creux (25a, 25b, 25c...) sont prévus, l'épaisseur de paroi du tube en verre (10) est constante le long de la circonférence du tube.

12. Dispositif selon l'une au moins des revendications précédentes, **caractérisé en ce que** la section du tube (10) est choisie de forme ronde, ovale, anguleuse, de préférence ronde ou ovale.

13. Dispositif selon l'une au moins des revendications précédentes, **caractérisé en ce que** les sources de lumière UV (20a, 20b, 20c...) sont disposées de façon symétrique dans le tube en verre (10).

14. Utilisation d'un dispositif selon l'une des revendications 1 à 13 pour désinfecter des liquides et/ou des gaz dans l'état statique ou en circulation, en particulier pour la préparation et la désinfection de l'eau potable, la désinfection d'eau extra-pure, de liquides dans l'industrie pharmaceutique ou alimentaire, pour la désinfection de gaz tels que l'air ou les gaz industriels ou similaires ainsi que dans la production d'eau extra-pure.
